# EUROPÄISCHE PATENTANMELDUNG

(11) **EP 1 479 390 A1**
(43) Veröffentlichungstag der Anmeldung: **24.11.2004**
(21) Anmeldenummer: 04001880.6
(22) Anmeldetag: 29.01.2004
(51) Int. Cl.: A61K 33/00

(54) **Arzneimittel mit Selenit**

(30) Priorität: 10.05.2003 DE 10321029
(71) Anmelder: Heide, Sigrid, 72147 Nehren (DE)
(72) Erfinder: Heide, Sigrid, 72147 Nehren (DE); Eichenauer, Johannes, Dr., 35644 Hohenahr (DE)
(74) Vertreter: Möbus, Daniela, Dr.-Ing.

(57) **Zusammenfassung**

Ein Arzneimittel mit einem Gehalt an Selenit, insbesondere Natrium- und/oder Kaliumselenit, und die Verwendung von Selenit und insbesondere von Natriumselenit zur Herstellung eines vorzugsweise in emulgierter, mikroverkapselter oder liposomaler Form vorliegenden Arzneimittels zur Behandlung von entzündlichen Erkrankungen.

## Beschreibung

Selen ist ein essentielles Spurenelement, d. h. der menschliche Körper benötigt dieses Element zum Erhalt lebenswichtiger Vorgänge und ist dabei auf eine externe Zufuhr angewiesen. Gemäß einer Empfehlung des US-National Research Council sollte die tägliche Zufuhr an Selen für Erwachsene 0,87 µg/kg Körpergewicht betragen. Die Aufnahme von Selen liegt in einem selenarmen Land wie Deutschland jedoch nur bei etwa 20 bis 50 µg pro Tag, sodass ein Erwachsener mit Selen latent unterversorgt ist. Die Gefahr des Selenmangels steigt mit dem Lebensalter, da die Fähigkeit zur Aufnahme des Spurenelements mit zunehmendem Alter nachlässt.

Selen wird daher in verschiedenen auf dem Markt erhältlichen Präparaten als Nahrungsergänzungsmittel bei Selenmangelzuständen angeboten. Die meisten Präparate sind Tabletten mit Hefen, die Selen und zusätzliche Nahrungsergänzungsstoffe wie Vitamine und Spurenelemente enthalten.

Der vorliegenden Erfindung liegt die Aufgabe zugrunde, ein Arzneimittel mit einem Selengehalt vorzuschlagen und dessen Anwendungsgebiete anzugeben. Die Aufgabe wird mit einem Arzneimittel mit einem Gehalt an Selenit, insbesondere an Natriumselenit und/oder Kaliumselenit, gelöst. Es hat sich herausgestellt, dass Selen und hier insbesondere das Natriumselenitsalz ein sehr guter Radikalfänger ist. Insofern ist dieser Wirkstoff insbesondere zur Behandlung von entzündlichen Prozessen einsetzbar.

Die Erfindung betrifft daher die Verwendung von Selenit, insbesondere von Natriumselenit und/oder Kaliumselenit, in verschiedenen Galeniken zur Behandlung von entzündlichen Erkrankungen.

Versuche haben ergeben, dass Selen und hier insbesondere das Natriumselenitsalz allen anderen Präparationen wie Vitaminen, Enzymen, Corticoiden, ungesättigten Fettsäuren usw. als Radikalfänger bei weitem überlegen ist. Dabei wirkt das Selen einerseits über die Glutathionperoxidase und andererseits als Selenit mit der Oxidationsstufe +4. Selenit kann bis zum Selenid (Oxidationsstufe -2) reduziert werden. Die bisherige Lehrmeinung sowohl der Biochemie als auch der Medizin stellt die Gluthationperoxidase als radikalhemmendes Enzym in den Vordergrund. Allerdings dauert die Neubildung dieses Enzyms mit Hilfe der Ribonukleinsäure Minuten. Natriumselenit dagegen entfaltet seine Wirkung sofort. In ionischer Form gelöst tritt seine zellprotektive Wirkung im Nano-Sekunden-Bereich ein. Dies liegt auch daran, dass das Dipolmoment des Natriumselenits den sehr niedrigen Wert von 0,68 Debye besitzt und somit Zellen und Gewebe in kurzer Zeit durchdringt.

Selenit, insbesondere Natriumselenit und/oder Kaliumselenit, kann in einer zur peroralen oder intravenösen Anwendung geeigneten Galenik zur Herstellung eines Arzneimittels zur Behandlung von entzündlichen Erkrankungen eingesetzt werden. Bei dieser Art der Anwendung entwickelt sich ein antioxidativer Schutz gegen membranschädliche Radikale wie z. B. Sauerstoffradikale, Hydroperoxide, Hydroxilradikale und dergleichen, die sich in den Zellen und im Plasma befinden. Diese ausgesprochen reaktiven Radikale haben ihren Ursprung entweder in endogenen oder exogenen Einflüssen. Ein Überangebot dieser Stoffe führt zum oxidativen Stress.

Als Beispiel für exogene Ursachen sind Einwirkungen von ultraviolettem Licht, von β- und Y-Strahlen, Laserlicht, Umwelteinflüsse wie Ozon, Stickoxide, Schwermetalle, der Gebrauch von Genussgiften, Nikotin und Alkohol, der Verzehr von geräucherten und gepökelten Lebensmitteln sowie tierischen Fetten und auch die Medikamenteneinnahme, insbesondere die Einnahme von Antibiotika und Zytostatika zu nennen.

Endogene Ursachen für oxidativen Stress sind Mangelernährung (Spurenelemente, Vitamine), Operationen und entzündliche Erkrankungen.

In allen diesen Fällen lässt sich Selen in Form von Selenit zur Reduktion des oxidativen Stresses einsetzen.

So kann Selenit, insbesondere Natriumselenit und/oder Kaliumselenit, in einer zur peroralen oder intravenösen Anwendung geeigneten Galenik zur Herstellung eines Arzneimittels zur Behandlung von Diabetes, Colitis, Morbus Crohn, Morbus Wilson, Arteriosklerose, Hypercholesterinämie, Niereninsuffizienz, Hyperhomocysteinämie, Schilddrüsenfunktionsstörungen, Mukoviscidose und/oder Allergien eingesetzt werden. Genauso kann Selenit in dieser Galenik zur Behandlung von Alzheimer, Parkinson, Hörsturz und Hirnschlag, entzündlichen Herzerkrankungen und zur Behandlung von Krebs, rheumatischen Erkrankungen und/oder genetischen Defekten eingesetzt werden. Diese Aufzählung von Krankheitsbildern ist jedoch nicht vollständig. Darüber hinaus entfaltet Selen nicht nur positive Eigenschaften als Radikalfänger, sondern auch günstige Wirkungen auf das Immunsystem, indem die Phagozytose und die Immunglobulinproduktion gesteigert wird. Selen wird auch zur Bildung der zurzeit 133 bekannten Selenproteine benötigt, die ihre Wirkung in verschiedenen Organen entfalten, u. a. in der Schilddrüse, der Leber, im Gehirn, in den Gonaden und in den endokrinen Organen.

Selenit und insbesondere Natriumselenit und/oder Kaliumselenit kann in einer zur topischen Anwendung geeigneten Galenik auch zur Behandlung von entzündlichen Erkrankungen der Haut, der Schleimhäute und/oder der Augen eingesetzt werden. Insbesondere Allergien, Neurodermitis, Psoriasis, Akne, Verbrennungen, Wunden, Melanome, Basaliome und/oder Hämorrhoiden können damit behandelt werden.

Durch pH-Verschiebung in ein saures Milieu entsteht aus der Natrium-Selenit-Verbindung selenige Säure, die etwas lipophiler ist und leichter in die Haut eindringt.

Das erfindungsgemäße Arzneimittel kann daher beispielsweise Selenit und insbesondere Natriumselenit in einer Wasser-in-Öl-Emulsion enthalten. Diese Cremes durchdringen die lipophile Hornschicht der Haut rasch. Der erhöhte Wassergehalt der tiefer gelegenen Stratum-corneum-Schichten bremst dagegen ein weiteres Eindringen der Wasser-in-Öl-Emulsion ab, sodass der Wirkstoff Selenit oder Natriumselenit längere Zeit im entzündlichen Bereich der Haut verweilen kann.

Das Arzneimittel kann jedoch auch einen Gehalt an Selenit-Liposomen aufweisen. Für diese Arzneimittel gibt es verschiedene Einsatzmöglichkeiten. Sie können als Spray auf die Haut aufgebracht werden und dort den Wirkstoff langsam und kontinuierlich freisetzen. Für den peroralen Gebrauch bzw. für die intravenöse Anwendung empfehlen sich liposomale Zubereitungen, bei denen Selenit entweder nur als Liposom oder als Mischung aus freier Lösung in Kombination mit Liposomen vorliegt. Die Selenit-Liposomen können auch so genannte Stealth-Liposomen sein. Diese Liposomen sind wegen ihrer negativen Ladung oder durch andere geeignete technologische Maßnahmen wie Pegylierung, Glycerol-Verbindung oder Tresyslierung mit Eigenschaften versehen, die verhindern, dass sie von den Makrophagen des Blutkreislaufs erkannt werden, sodass sie länger im Kreislauf verbleiben. Solche Arzneimittel eignen sich insbesondere bei Krankheitsbildern wie Colitis oder Morbus Crohn, bei denen eine reine Selenit-Lösung wenig helfen würde, da das Selenit zu rasch über Harn bzw. Faeces ausgeschieden wird.

Bei bestimmten Erkrankungen der Milz und der Leber oder Galle ist dagegen die Phagozytose der Selenit-Liposomen erwünscht, sodass vorzugsweise unbehandelte Selenit-Liposomen verwendet werden. Diese Liposomen werden von den Makrophagen markiert und im Bereich der Milz und/oder Leber angesammelt. Anschließend werden die opsonisierten Selenit-Liposomen in der Leber aufgeschlossen, wobei der Wirkstoff Natriumselenit oder Kaliumselenit in diesem Organ wie erwünscht freigesetzt wird.

Die Selenit-Liposomen können auch mit Antikörpern angereichert sein, sodass diese Liposomen gezielt zu den entsprechenden Antigenen des betroffenen Organs oder Gewebes wandern und sich dort anreichern.

Das Arzneimittel kann auch in Form eines Inhalats eingesetzt werden. Es ist dann insbesondere zur Behandlung von Asthma Bronchiale geeignet. Zusätzlich kann Natriumselenit und/oder Kaliumselenit auch als Trinklösung eingesetzt werden. Damit kann die bisher bei Asthma übliche Cortisontherapie eingeschränkt bzw. ganz vermieden werden. Inhalate mit Selenit-Liposomen haben den Vorteil, dass die Lecithine der Liposomen chemisch stark mit den Lecithinen der Lungenflüssigkeit verwandt sind, sodass die Funktion der Lungenbläschen unterstützt wird.

Das Arzneimittel kann auch in Form von Tropfen zur äußeren Anwendung, insbesondere in Form von Augentropfen, vorliegen. Dabei können diese Tropfen mit oder ohne Liposomen ausgestattet sein. Das Selenit kann jedoch auch in Globuli oder in Puder eingebunden werden. Auch eine Verwendung in Form von Ohrentropfen, Nasentropfen oder Nasenspray ist möglich.

Weitere Vorteile ergeben sich, wenn das Arzneimittel auf einem Trägermaterial, beispielsweise auf Suppositorien, Klysmen, Tamponaten, Pflastern oder transdermalen Systemen aufgebracht ist. Die Wirkstoffabgabe kann dann sehr gezielt und lang andauernd erfolgen.

Natriumselenit ist als Nahrungsergänzungsmittel Bestandteil der Präparate SELENASE (Fa. Biosyn) und CEFASEL (Fa. Cefak). Es werden in diesen Präparaten das Natriumselenit als Lösung oder in fester Form als Tabletten bzw. Kapseln angeboten.

Veränderte Ernährungsgewohnheiten und die nachhaltige Intensivierung der Lebensmittelproduktion in den Industrieländern trägt dazu bei, dass ein Großteil der in den Lebensmittelballaststoffen ursprünglich vorhandenen Makro- und Mikronährstoffe unzureichend dem menschlichen bzw. tierischen Organismus zugeführt wird. Es besteht ein Zusammenhang zwischen der gesunkenen Versorgung mit Makro- und Mikronährstoffen und einer Vielzahl von Stoffwechselkrankheiten und Störungen des Immunsystems gerade in den Industrieländern. Die beginnende Unterversorgung mit den Elementen Calcium, Magnesium, Kalium, Kupfer, Eisen, Zink, Selen, Jod und Fluor erfolgt zunächst ohne Anzeichen von Symptomen. Über längere Zeit hat aber diese Unterversorgung gerade in Stresssituationen (gesundheitlicher, körperlicher und seelischer Natur) eine negative Wirkung auf zellphysiologische Abläufe, die durch ein kurzzeitig erhöhtes Nährstoffangebot nicht ausgeglichen werden können. Eine dauerhaft erhöhte Zufuhr dieser Makro- und Mikronährstoffe führt zunächst zum Auffüllen des Körperdepots, birgt aber gerade bei den wasserlöslichen Stoffen die Gefahr in sich, dass eine kurzfristige Überversorgung mit nachhaltigen Nebenwirkungen und Imbalancen induziert werden kann.

Eine umfangreiche Bedeutung besitzen liposomale und mikroverkapselte Selenverbindungen sowie andere Radikalfänger (Vitamine, Enzyme, Aminosäuren) in der medizinischen Prävention bei SIRS (systemic inflammatory respons syndrom) oder einer Sepsis, die bei medizinischen Eingriffen auftreten kann. Aufgrund der zeitlich längeren und damit gleichmäßigeren Wirkungsweise sind diese Produkte eher geeignet, einen Mangelzustand zu beheben ohne eine kurzfristige Überdosierung mit toxischen Reaktionen zu induzieren.

Die enge Bandbreite der optimalen Selenversorgung soll hier aufgeführt werden:

Unterversorgung liegt vor bei einem Selen-Gehalt von <89 µg/l Vollblut. Optimal liegt der Selengehalt bei 89 - 168 µg/l Vollblut. Wir sprechen von einem erhöhten aber klinisch unbedenklichen Selengehalt bei Werten von 169 - 230 µg/l Vollblut. Kontrollen sind unbedingt nötig bei Selenkonzentrationen >230 µg/l Vollblut.

Als Neuheit für Nahrungsergänzungsmittel wird Natrium- bzw. Kaliumselenit erfindungsgemäß in Liposomen- und in mikroverkapselter Form bzw. eine Kombination aus Natrium- bzw. Kaliumselenit vermischt in reiner und liposomaler bzw. mikroverkapselter Art vorgestellt.

Natrium- bzw. Kaliumselenit ist in der Lage, nach Aufnahme in den Körper sofort in den menschlichen bzw. tierischen Zellen seine Wirkung zu entfalten, da wegen des niedrigen Dipolmoments das Eindringen in die Zelle erleichtert wird. Der extrazelluläre Raum enthält Natrium-Ionen, der intrazelluläre hingegen viele Kalium-Ionen. Es ist deshalb von Vorteil, dem Körper auch Kaliumselenit anzubieten, da dieses Elektrolyt rasch in das Zellinnere gelangt. Um einen schnellen Abbau und damit das rasche Eliminieren von Selenverbindungen über Harn, Faeces, Haut und Lunge zu verzögern, sollte zumindest ein Anteil der Natrium-/Kalium-Selenite in Liposomenform und/oder mikroverkapselt vorliegen.

Durch die rasche Reaktion der Selenite sowohl bei der Aufnahme in den Körper als auch bei der Metabolisierung und damit beim Ausscheiden aus dem menschlichen bzw. tierischen Körper bedeutet dies nach einer gewissen Zeit, dass sich der Selenspiegel erniedrigt.

Dies kann man dadurch umgehen, indem man eine erneute Dosis Selenit zuführt mit der Gefahr der Überdosierung oder erfindungsgemäß einen Teil der Selenite in Liposomen-Mikrokapseln überführt. Diese Liposomen haben einen Depoteffekt und geben die eingeschlossenen Selenite allmählich ab. Die besondere Eigenschaft der Liposomen aus Lecithin ist es, dass sie sich auch an körpereigene Eiweißmoleküle anlagern können, die an ihrer Oberfläche eher hydrophobe Regionen aufweisen. Die Freigabe aus Mikrokapseln kann zeitlich durch verschiedene Wandstärken der Kapseln oder aber auch durch pH-Verschiebung gesteuert werden. Celluloseacetatphthalat- oder auch Polyacrylat-Lacke (EUDRAGIT ^{TM}) sind z. B. magensaftresistent und lösen sich erst im alkalischen Milieu des Dünndarms auf. Neueste physikalisch-chemische Untersuchungen beweisen, dass selbst elementares, molekulardisperses Selen (rote Form) in den Mitochodrien der Zellen antioxidative Prozesse quantenphysikalisch messbar auslöst.

Aus der Kenntnis der geringen Bandbreite zwischen Über- und Unterversorgung der Makro- und Mikronährstoffe werden eine Reihe dieser Elemente in organischen Verbindungen eingesetzt und dem Körper zugeführt. Diese Formulierungen besitzen zwar ein geringeres Risiko der akuten Überdosierung, da sie aber den hepatischen Kreislauf mehrmals durchlaufen müssen, bis das Spurenelement in physiologisch verfügbarer Form vorliegt, ist hier die Gefahr der Vergiftung höher. Die erfindungsgemäße Anwendung von Selen, Selensalzen in freier und liposomaler bzw. mikroverkapselter Form führt zu optimalen Selenspiegeln.

Eine zu kompensierende Selen-Unterversorgung ist zunächst symptomlos, aber es besteht ein erhöhtes Risiko, nachfolgende Erkrankungen zu erleiden: Systemische und topische Entzündungen und Infektionen, Erkrankungen der Schilddrüse, der Milz, Leber, Galle, Pankreas, Dünn- und Dickdarm, der Nieren, der Prostata, Fertilitätsstörung, der Bauchspeicheldrüse, verschiedenste Krebserkrankungen wie z. B. Melanome, Sarkome, Basaliome, Leukämie, ferner Arteriosklerose, Rheuma, Asthma, Infarkte, diverse neuronale Erkrankungen, Epilepsie, Alzheimer, Parkinson, Hörsturz, Multiple Sklerose; diverse entzündliche Hauterkrankungen wie Neurodermitis, Psoriasis, Rosacea, Akne, Sonnenbrand, Verbrennungen, Allergien; Hautschäden hervorgerufen durch Viren, Insekten, Quallen; Stomatitis, Gingivitis, Parodontitis; Erkrankungen des Auges wie grüner und grauer Star, Makuladegeneration, Kontaktallergie. Diesen Erkrankungen kann durch die erfindungsgemäße Verabreichung eines Mittels mit einem Gehalt an Selenit, insbesondere Natrium- und/oder Kaliumselenit, vorzugsweise in emulgierter, mikroverkapselter oder liposomaler Form vorgebeugt oder entgegengewirkt werden.

Unterstützend wirkt das erfindungsgemäße Mittel bei Patienten mit parenteraler Ernährung, bei streng vegetarischer Ernährung, beim DialysePatienten, bei Schwermetallbelastung (z. B. Amalgam), bei Genussmittelmissbrauch, während der Schwangerschaft und der Stillzeit.

Es können auch essentielle Mikronährstoffe erfindungsgemäß kombiniert werden mit bedeutenden Makronährstoffen wie Kaliumselenit in isotonen Lösungen zur Stabilisierung des Flüssigkeitshaushalts.

Eigene klinische Untersuchen haben gezeigt, dass Pflegepräparate mit Natriumselenit, Kaliumselenit und vor allem ihre liposomalen wie auch Mikroemulsionszubereitungen hautschützend wirken.

Entzündliche Prozesse wurden durch Anwendung selenithaltiger externer Präparationen gemildert oder gänzlich unterbunden. Auch tiefer in der Haut platzierte Entzündungsherde konnten mit diesen Zubereitungen erreicht werden. Die Lipidoxidbildung der Hautfette wird reduziert, sodass die Hautalterung gemindert wird. Durch Immunstimulation über die Langerhanszellen in der Corneozytenschicht wird die Schutzfunktion der Haut verbessert.

Ferner empfiehlt sich die Zubereitung von Natrium-/Kaliumselenit auch als liposomale Zubereitung in der Landwirtschaft als Fütterungsmittel und als Düngemittel. Durch das Überdüngen der mitteleuropäischen Agrarflächen seit Beginn der 50er-Jahre verarmen diese Böden in ihrem Selengehalt, sodass Getreide und Futtermittel konstant niedrige Selenwerte aufweisen. Mensch und Tier nehmen also niedrige Mengen des essentiellen Minerals Selen über die Nahrungskette auf mit den bekannten Folgeerscheinungen des Selenmangels.

Als Düngemittel kann Selenit in Form von Pellets mikroverkapselt auf Felder und Weideflächen aufgebracht werden. Der Vorteil dieser Methode ist es, dass keine Selenit-Elektrolyte sofort mit den "sauren" Böden reagieren und inaktiviert werden, sondern das zeitliche Freisetzen aus der Mikrokapselhülle durch natürliche oder künstliche Umgebungsparameter wie Feuchtigkeit, Licht und Temperatur gesteuert werden kann. Diese Mikrokapselhüllen können aus natürlichen Materialien wie Cellulose, Alginate, Gelatine, Lecithinen sowie in Kombinationen mit diesen Stoffen bestehen.

## Patentansprüche

1. Arzneimittel **gekennzeichnet durch** einen Gehalt an Selenit, insbesondere Natrium- und/oder Kaliumselenit.

2. Arzneimittel nach Anspruch 1, **dadurch gekennzeichnet, dass** das Selenit zumindest teilweise in mikroverkapselter Form vorliegt.

3. Arzneimittel nach Anspruch 1 oder 2, **dadurch gekennzeichnet, dass** es einen Gehalt an Selenit-Liposomen aufweist.

4. Arzneimittel nach Anspruch 3, **dadurch gekennzeichnet, dass** die Selenit-Liposomen Stealth-Liposomen sind.

5. Arzneimittel nach Anspruch 3, **dadurch gekennzeichnet, dass** die Selenit-Liposomen mit Antikörpern angereichert sind.

6. Arzneimittel nach einem der Ansprüche 1 bis 5, **dadurch gekennzeichnet, dass** es als Inhalat anwendbar ist.

7. Arzneimittel nach einem der Ansprüche 1 bis 5, **dadurch gekennzeichnet, dass** es als Tropfen zur äußeren Anwendung, insbesondere als Augentropfen, verwendbar ist.

8. Arzneimittel nach einem der Ansprüche 1 bis 5, **dadurch gekennzeichnet, dass** es auf die Haut aufsprühbar ist.

9. Arzneimittel nach einem der Ansprüche 1 bis 5, **dadurch gekennzeichnet, dass** es in Form von Globuli vorliegt.

10. Arzneimittel nach Anspruch 1, **dadurch gekennzeichnet, dass** es einen Gehalt an Selenit in einer Wasser-in-Öl-Emulsion oder Öl-in-Wasser-Emulsion aufweist.

11. Arzneimittel nach Anspruch 1, **dadurch gekennzeichnet, dass** es als Mikroemulsion, Gel, Xerogel, Salbe, Creme, Lösung, Granulat, Pulver oder Aerosol vorliegt.

12. Arzneimittel nach einem der Ansprüche 1 bis 5 oder 10, **dadurch gekennzeichnet, dass** es auf einen Träger aufgebracht ist.

13. Arzneimittel nach Anspruch 12, **dadurch gekennzeichnet, dass** der Träger ein Suppositorium, ein Tampon oder ein Pflaster ist.

14. Verwendung von Selenit, insbesondere von Natrium- und/oder Kaliumselenit, in geeigneten Galeniken zur Herstellung eines Arzneimittels zur Behandlung von entzündlichen Erkrankungen.

15. Verwendung nach Anspruch 14 in einer zur peroralen oder intravenösen Anwendung geeigneten Galenik zur Herstellung eines Arzneimittels zur Behandlung von entzündlichen Erkrankungen.

16. Verwendung nach Anspruch 15 zur Behandlung von Diabetes, Colitis, Morbus Crohn, Morbus Wilson, Arteriosklerose, Hypercholesterinämie, Niereninsuffizienz, Hyperhomocysteinämie, Schilddrüsenfunktionsstörungen, Herzerkrankungen, Mukoviscidose, Allergien, Alzheimer, Parkinson, Hörsturz, Hirnschlag, Krebs, rheumatischen Erkrankungen und/oder genetischen Defekten.

17. Verwendung von Selenit, insbesondere von Natrium- und/oder Kaliumselenit, in einer zur topischen Anwendung geeigneten Galenik zur Behandlung von entzündlichen Erkrankungen der Haut, der Schleimhäute und/oder der Augen, insbesondere zur Behandlung von Allergien, Neurodermitis, Psoriasis, Akne, Verbrennungen, Wunden, Melanomen, Basaliomen und Hämorrhoiden.
